# EUROPEAN PATENT APPLICATION

(11) **EP 4 806 620 A1**
(43) Date of publication of application: **16.09.2026**
(21) Application number: 26163185.7
(22) Date of filing: 09.03.2026
(51) Int. Cl.: B29C 65/08, B29L 31/00, B29C 65/18, B29L 31/48

(54) **ROTARY APPARATUS FOR SEALING A CONTINUOUS WEB AND RELATED METHOD**

(30) Priority: 13.03.2025 IT 202500005196
(71) Applicant: GDM S.p.A., 40133 Bologna (IT)
(72) Inventor: ZAVALLONI, Alessandro, 40133 Bologna (IT); LISSANI, Gino, 40133 Bologna (IT)
(74) Representative: Emmi, Mario

(57) **Abstract**

A rotary apparatus (1) for sealing a continuous web (2) and related method, preferably for making absorbent articles or for producing containment bags of liquid or solid products; said apparatus comprises a first member (3) rotating according to a first direction (V') about an axis of rotation (3a) thereof, one or more first support elements (4) of said continuous web (2) supported by said first rotary member (3) and at least one first sealing unit (5') of the continuous web (2) supported by said first rotary member (3),
said rotary apparatus (1) furthermore comprising a second member (6) rotating according to a second direction (V") and rotating about an axis of rotation (6a) thereof, one or more second support elements (7) of said continuous web (2) supported by said second rotary member (6) and at least one second sealing unit (5") of the continuous web (2) supported by said second rotary member (6),
said first sealing unit (5') being configured to produce at least one first seal on said continuous web (2) and said second sealing unit (5") being configured to produce at least one second seal on said continuous web (2).

## Description

### Technical field of the invention:

The present invention relates to a rotary sealing apparatus of a continuous web.

In particular, the apparatus according to the present invention is configured to seal such a web preferably in a transverse direction, with respect to a forward direction of the web, according to a certain sealing pitch.

Purely by way of non-limiting example, said apparatus finds advantageous application in the production of absorbent articles or for the production of containment bags for liquid or solid products.

Preferably, said absorbent products comprise nappies, adult diapers, sanitary pads, drawsheets and plasters.

Moreover, purely by way of non-limiting example, said apparatus finds advantageous application in the production of containment bags for liquid or solid products for the food, pharmaceutical and tobacco industries.

The present invention also relates to a method of sealing a continuous web.

In particular, the method according to the present invention is configured to seal such a web preferably in a transverse direction, with respect to a forward direction of the web, according to a certain sealing pitch.

Purely by way of non-limiting example, said method finds advantageous application in the production of absorbent articles or for the production of containment bags for liquid or solid products.

Preferably, said absorbent products comprise nappies, adult diapers, sanitary pads, drawsheets and plasters.

Moreover, purely by way of non-limiting example, said method finds advantageous application in the production of containment bags for liquid or solid products for the food, pharmaceutical and tobacco industries.

### State of the art:

As is well known, in recent years, technological development for the production of products on an industrial scale by means of automatic machines, in particular for the production of absorbent articles (e.g. nappies, diapers, sanitary pads, drawsheets and plasters) or for the production of bags for containing liquid or solid products (e.g. for the food industry, pharmaceutical and tobacco industries), focused on making consecutive seals on a web (continuous or discrete) of material according to a given pitch and along a forward direction of the web itself or transverse to said forward direction.

In particular, rotary sealing apparatuses are known which involve the use of a rotary member supporting a plurality of sealing units configured to perform continuous web sealing while the rotary member is rotating about its axis of rotation.

The radial positioning of the sealing units, with respect to the axis of rotation of the rotary member, defines a certain working circumference of the sealing units, defining a respective sealing pitch.

In order to vary the sealing pitch of the device, in the event of a product change, size change or brand change, it is necessary to either use a rotary member with all the necessary sealing units or to vary the radial positioning of the sealing units, with respect to the axis of rotation of the rotary member, so as to determine the working circumference along which the desired sealing pitch is obtained.

However, said apparatuses have several drawbacks.

A first drawback is the fact that, in the event of a product change or size change or brand change where the pitch (distance) between two consecutive seals is "reduced" (due to the intrinsic dimensions of the product to be produced), it is not possible to bring the sealing units radially close enough due to the mechanical interference generated between the sealing units themselves, which severely limits the production flexibility of said sealing apparatuses.

A second drawback is the fact that said sealing apparatuses are unable to guarantee a continuous product change or size change or brand change, i.e. according to any product size parameter, due to the aforementioned mechanical interference that is generated between the sealing units themselves, again severely limiting the production flexibility of said sealing apparatuses.

Furthermore, a third drawback is the fact that said systems involve the use of a single rotary apparatus to make said seals on said web, which potentially affects the productivity of the entire production line in the event of the malfunction of even one sealing unit, since the rotary apparatus is required to be shut down in order to replace said malfunctioning sealing unit, which implies the shutdown of the entire production line.

### Summary of the invention:

The object of the present invention is to provide a rotary sealing apparatus for sealing a continuous web, preferably for making absorbent articles or for making bags for containing liquid or solid products, which overcomes the above-mentioned drawbacks of the prior art.

In particular, it is the object of the present invention to provide a rotary sealing apparatus for sealing a continuous web, preferably for making absorbent articles or for making bags for containing liquid or solid products, which allows high production flexibility and high productivity.

Furthermore, it is the object of the present invention to provide a method of sealing a continuous web, preferably for the production of absorbent articles or for the production of bags for the containment of liquid or solid products, which overcomes the drawbacks of the above-mentioned prior art.

In particular, it is the object of the present invention to make available a method of sealing a continuous web, preferably for the production of absorbent articles or for the production of bags for the containment of liquid or solid products, which allows high production flexibility and high productivity.

According to the present invention, a rotary sealing apparatus 1 is provided for sealing a continuous web, preferably for making absorbent articles or for making bags for containing liquid or solid products, as defined in claim 1.

Purely by way of non-limiting example, said apparatus finds advantageous application in the production of absorbent articles or for the production of containment bags for liquid or solid products.

Preferably, said absorbent products comprise nappies, adult diapers, sanitary pads, drawsheets and plasters.

Moreover, purely by way of non-limiting example, said apparatus finds advantageous application in the production of containment bags for liquid or solid products for the food, pharmaceutical and tobacco industries.

Preferably, said apparatus comprises a first rotary member 3.

The term rotary member 3 refers to any member rotating in a respective direction (in this case, first direction V') about its own axis of rotation (in this case, 3a).

Preferably, said apparatus 1 comprises one or more first support elements 4 of said continuous web 2.

The term support element 4 refers to any element configured to support said continuous web 2.

Preferably, said one or more first support elements 4 are supported by said first rotary member 3.

Preferably, said apparatus 1 comprises at least one first sealing unit 5' for sealing the continuous web 2.

Preferably, said at least one first sealing unit is supported by said first rotary member 3.

Preferably, said rotary apparatus 1 further comprises a second member 6 rotating according to a second direction (V") and rotating about its own axis of rotation 6a.

Preferably, said apparatus comprises one or more second support elements 7 of said continuous web 2.

Preferably, said one or more support elements are supported by said second rotary member 6.

Preferably, said apparatus comprises at least one second sealing unit 5" for sealing the continuous web 2.

Preferably, said at least one second sealing unit is supported by said second rotary member 6.

Preferably, said first sealing unit 5' is configured to produce at least one first seal on said continuous web 2.

Preferably, said second sealing unit 5" is configured to perform at least one second seal on said continuous web 2.

Said at least one first seal and said at least one second seal can be made at the same sealing point, essentially overlapping each other.

In addition or alternatively, said at least one first seal and said at least one second seal can be produced at different sealing points, e.g. contiguous according to a certain pitch, substantially not overlapping each other.

This solution solves all the aforementioned technical problems.

In particular, the presence of said first rotary member 3 and of said second rotary member 6 with respective first sealing unit 5' and second sealing unit 5", respectively configured to perform at least one first seal and at least one second seal on said continuous web 2, allows to ensure high production flexibility and high productivity, since it avoids the aforementioned mechanical interference between sealing units of the same rotary member or adjacent rotary members, as well as the possibility of avoiding downtime in case of malfunction of at least one sealing unit, as it is possible to stop only one rotary member (supporting the malfunctioning sealing unit) while the further rotary member remains active.

According to the present invention, a method for sealing a continuous web is also provided, preferably for making absorbent articles or for making bags for containing liquid or solid products, as defined in claim 11.

Purely by way of non-limiting example, said method finds advantageous application in the production of absorbent articles or for the production of containment bags for liquid or solid products.

Preferably, said absorbent products comprise nappies, adult diapers, sanitary pads, drawsheets and plasters.

Moreover, purely by way of non-limiting example, said method finds advantageous application in the production of containment bags for liquid or solid products for the food, pharmaceutical and tobacco industries.

Preferably, said method comprises a step of providing a first rotary member 3.

The term rotary member 3 refers to any member rotating in a respective direction (in this case, first direction V') about its own axis of rotation (in this case, 3a).

Preferably, said method comprises a step of providing one or more first support elements 4 of said continuous web 2.

The term support element 4 refers to any element configured to support said continuous web 2.

Preferably, said one or more first support elements 4 are supported by said first rotary member 3.

Preferably, said method comprises a step of providing at least one first sealing unit 5' for sealing the continuous web 2.

Preferably, said at least one first sealing unit is supported by said first rotary member 3.

Preferably, said method further comprises a step of providing a second member 6 rotating according to a second direction (V") and rotating about its own axis of rotation 6a.

Preferably, said method comprises a step of providing one or more second support elements 7 of said continuous web 2.

Preferably, said one or more support elements are supported by said second rotary member 6.

Preferably, said method comprises a step of providing at least one second sealing unit 5" for sealing the continuous web 2.

Preferably, said at least one second sealing unit is supported by said second rotary member 6.

Preferably, said first sealing unit 5' is configured to produce at least one first seal on said continuous web 2.

Preferably, said second sealing unit 5" is configured to perform at least one second seal on said continuous web 2.

Said at least one first seal and said at least one second seal can be made at the same sealing point, essentially overlapping each other.

In addition or alternatively, said at least one first seal and said at least one second seal can be produced at different sealing points, e.g. contiguous according to a certain pitch, substantially not overlapping each other.

This solution solves all the aforementioned technical problems.

In particular, the presence of said first rotary member 3 and of said second rotary member 6 with respective first sealing unit 5' and second sealing unit 5", respectively configured to perform at least one first seal and at least one second seal on said continuous web 2, allows to ensure high production flexibility and high productivity, since it avoids the aforementioned mechanical interference between sealing units of the same rotary member or adjacent rotary members, as well as the possibility of avoiding downtime in case of malfunction of at least one sealing unit, as it is possible to stop only one rotary member (supporting the malfunctioning sealing unit) while the further rotary member remains active.

### Brief description of the drawings:

Further features and advantages of the present invention will become apparent from the detailed description that follows, provided by way of non-limiting example with reference to the accompanying drawings, wherein:
Figure 1 shows a schematic view of a rotary sealing apparatus of a continuous web, according to the invention;
Figure 2 shows a schematic view of possible configurations for said at least one first seal and said at least one second seal on said continuous web, according to the invention.

### Detailed description of the invention:

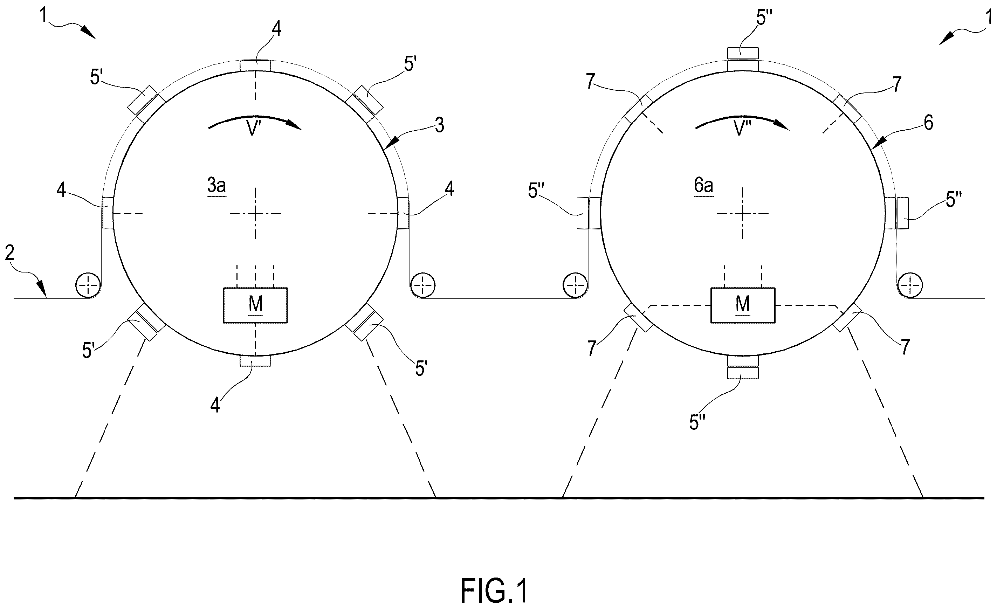

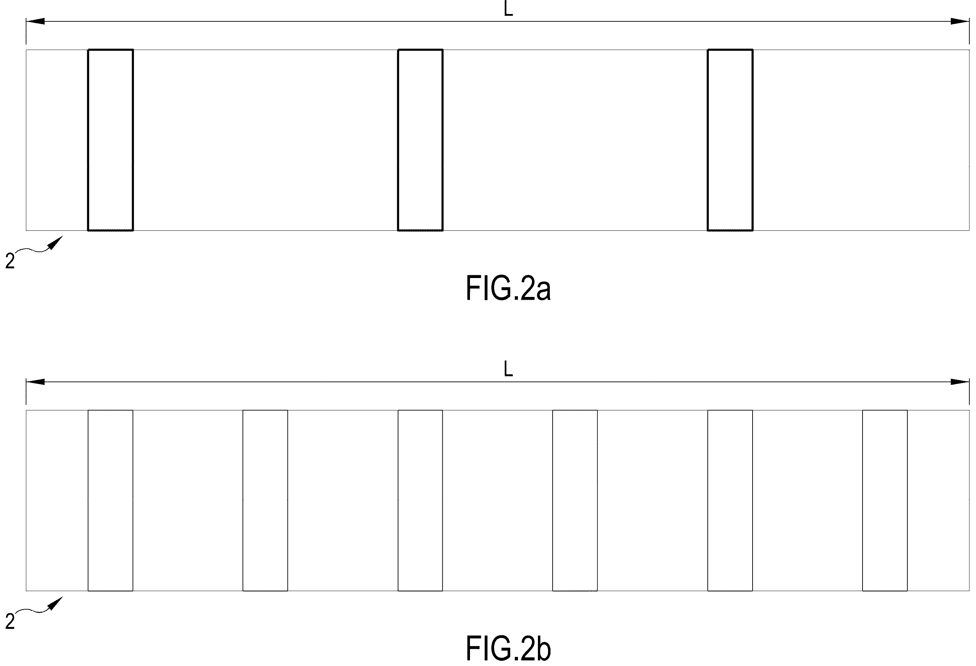

Referring to Figure 1 and Figure 2, purely by way of non-limiting example, said apparatus finds advantageous application in the production of absorbent articles or for the production of containment bags for liquid or solid products.

Preferably, said absorbent products comprise nappies, adult diapers, sanitary pads, drawsheets and plasters.

Moreover, purely by way of non-limiting example, said apparatus finds advantageous application in the production of containment bags for liquid or solid products for the food, pharmaceutical and tobacco industries.

Preferably, said apparatus comprises a first rotary member 3.

The term rotary member 3 refers to any member rotating in a respective direction (in this case, first direction V') about its own axis of rotation (in this case, 3a).

Preferably, said apparatus 1 comprises one or more first support elements 4 of said continuous web 2.

The term support element 4 refers to any element configured to support said continuous web 2.

Preferably, said one or more first support elements 4 are supported by said first rotary member 3.

Preferably, said apparatus 1 comprises at least one first sealing unit 5' for sealing the continuous web 2.

Preferably, said at least one first sealing unit is supported by said first rotary member 3.

Preferably, said rotary apparatus 1 further comprises a second member 6 rotating according to a second direction (V") and rotating about its own axis of rotation 6a.

Preferably, said apparatus comprises one or more second support elements 7 of said continuous web 2.

Preferably, said one or more support elements are supported by said second rotary member 6.

Preferably, said apparatus comprises at least one second sealing unit 5" for sealing the continuous web 2.

Preferably, said at least one second sealing unit is supported by said second rotary member 6.

Preferably, said first sealing unit 5' is configured to produce at least one first seal on said continuous web 2.

Preferably, said second sealing unit 5" is configured to perform at least one second seal on said continuous web 2.

Said at least one first seal and said at least one second seal can be made at the same sealing point, substantially overlapping each other.

In addition or alternatively, said at least one first seal and said at least one second seal can be produced at different sealing points, e.g. contiguous according to a certain pitch, substantially not overlapping each other.

These characteristics derive from the experimental tests carried out by the Applicant, in which the Applicant tried out different alternative solutions, in order to find the solution that best guaranteed high production flexibility and productivity.

In particular, the presence of said first rotary member 3 and of said second rotary member 6 with respective first sealing unit 5' and second sealing unit 5", respectively configured to perform at least one first seal and at least one second seal on said continuous web 2, allows to ensure high production flexibility and high productivity, since it avoids mechanical interference between sealing units of the same rotary member or adjacent rotary members, as well as the possibility of avoiding downtime in case of malfunction of at least one sealing unit, as it is possible to stop only one rotary member (supporting the malfunctioning sealing unit) while the further rotary member remains active.

Furthermore, in the event that said at least one first seal and said at least one second seal are performed at the same sealing point, substantially overlapping each other (in addition to or alternatively to being performed at different sealing points), the apparatus ensures high productivity; this is because, if one of said at least one first seal and said at least one second seal is performed according to sealing parameters that are lower than those required and predetermined (due, for example, to malfunctioning of a respective sealing unit), the other of said at least one first seal and said at least one second seal compensates for such failure and makes it possible to avoid rejection of the respective end product and/or stoppage of the respective rotary member (and of the production line).

According to a further aspect of the present invention, said first sealing units 5' and said second sealing units 5" are arranged respectively on said first rotary member 3 and said second rotary member 6 in such a manner that said first sealing units 5" and said second sealing units 5" can respectively perform in an alternating and complementary manner the seals provided on said continuous web 2.

In other words, said first sealing units 5' are arranged on said first rotary member 3 according to a predetermined configuration to produce said at least one first seal on said continuous web 2, while said second sealing units 5" are arranged on said second member 6 rotating according to a predetermined complementary configuration (i.e., alternating) with respect to the configuration for said first sealing units 5' so as to produce said at least one second seal on said continuous web 2 at said at least one first seal (as shown in Figure 2a) or at a sealing point contiguous to said at least one first seal (as shown in Figure 2b).

In this way, each rotary member has fewer (preferably, half the number of) sealing units required to produce said at least one first and at least one second sealing unit on said continuous web 2.

These characteristics derive from experimental tests carried out by the Applicant, wherein the Applicant tried out different alternative solutions, in order to identify the solution that best ensured high production flexibility and productivity.

In particular, this configuration for said first sealing units 5' and said second sealing units 5" ensures high production flexibility and high productivity, since it avoids mechanical interference between the sealing units of said rotary members, as well as the possibility of avoiding downtime in the event of malfunction of at least one sealing unit, since only one rotary member (supporting the malfunctioning sealing unit) can be stopped while the further rotary member remains active.

Furthermore, in the event that said at least one first seal and said at least one second seal are performed at the same sealing point, substantially overlapping each other (in addition to or alternatively to being performed at different sealing points), the apparatus ensures high productivity; this is because, if one of said at least one first seal and said at least one second seal is performed according to sealing parameters that are lower than those required and predetermined (due, for example, to malfunctioning of a respective sealing unit), the other of said at least one first seal and said at least one second seal compensates for such failure and makes it possible to avoid rejection of the respective end product and/or stoppage of the respective rotary member (and of the production line).

According to a further aspect of the present invention, each of said first sealing units 5' and said second sealing units 5" operates according to a pitch (i.e., a distance) equal to at least two products to be made on said continuous web 2.

This configuration for said first sealing units 5' and said second sealing units 5" ensures high production flexibility and high productivity, as it avoids mechanical interference between the sealing units of said rotary members.

According to a further aspect of the present invention, said at least one first seal and at least one second seal are made transverse to a long side L of said continuous web 2, as shown in Figure 2.

In particular, said transverse sealing is of the side seam bonding type.

According to a further aspect of the present invention, said continuous web 2 comprises at least one component of a sanitary absorbent item wearable as panties.

In other words, from said continuous web 2, by means of appropriate processing including said at least one first and at least one second seal, at least one component is obtained for the production of a sanitary absorbent item wearable as panties, preferably a pull-up nappy.

According to a further aspect of the present invention, said first sealing units 5' and/or said second sealing units 5" comprise an ultrasound sealing device or a thermomechanical sealing device or a thermal sealing device.

According to a further aspect of the present invention, said apparatus 1 comprises actuation means M configured to vary a pitch defined by each of the one or more first support elements 4 and/or the one or more second support elements 7, expanding or contracting the working circumference of the first and/or second sealing units 5', 5".

In other words, said actuation means M are configured to radially vary the sealing pitch by acting on the mutual spacing between said one or more first support elements 4.

Alternatively or additionally, such actuation means M are configured to vary the sealing pitch radially by acting on the mutual spacing between said one or more second support elements 7.

Substantially, by means of said actuation means M, the diameter of the rotary members (3a, 6a) can be changed and/or the size of the support elements (4, 7) on which the web being processed rests can be modified.

This feature provides a high degree of production flexibility, as it avoids mechanical interference between the sealing units of said rotary members, allowing for continuous product change, size change or brand change.

According to a further aspect of the present invention, said actuation means M comprise a control and command unit configured to adjust at least one mechanical element comprised by said one or more first support elements 4 and/or said one or more second support elements 7.

In other words, said control and command unit of said actuation means M comprises any automatic or semi-automatic technical solution configured to radially vary the sealing pitch by acting on the mutual spacing between said one or more first support elements 4.

Alternatively or additionally, said control and command unit of said actuation means M comprises any automatic or semi-automatic technical solution configured to vary the sealing pitch radially by acting on the mutual spacing between said one or more second support elements 7.

This feature provides a high degree of production flexibility, as it avoids mechanical interference between the sealing units of said rotary members, allowing the product change, size change or brand change to be carried out continuously and efficiently, as no adjustments made manually by an operator are required (which could lead to problems in the event of human error).

According to a further aspect of the present invention, said apparatus comprises holding means configured to hold said continuous web 2 on said one or more first support elements 4 and/or one or more second support elements 7.

Preferably, said holding means comprise a belt and/or pneumatic vacuum.

Said characteristic ensures a high production efficiency, since by means of said holding means the continuous web 2 remains optimally positioned on said one or more first support elements 4 and/or said one or more second support elements 7, avoiding misplacements due to the high rotation speed of said at least one of said rotary members; thus, said first sealing units 5' and said second sealing units 5" can accurately and reproducibly provide said at least one first seal and said at least one second seal on said continuous web 2.

According to a further aspect of the present invention, said second rotary member 6 is arranged downstream of said first rotary member 3 and said second direction V" is concordant with said first direction V'.

In other words, said second rotary member 6 is arranged downstream of said first rotary member 3 along said forward direction of the continuous web 2. Said continuous web 2 has a direction in which it traverses at least one section of said first rotary member 3 and is defined by said first direction V', and said continuous web 2 has a direction in which it traverses at least one section of said second rotary member 6 and is defined by said second direction V". In the event that said first direction V' and said second direction V" are concordant, said continuous web 2 travels along said first rotary member 3 and said second rotary member along the forward direction of said continuous web 2.

According to an alternative embodiment not illustrated, said second rotary member 6 is arranged downstream of said first rotary member 3 and said second direction V" is opposite to said first direction V'.

In other words, said second rotary member 6 is arranged downstream of said first rotary member 3 along said forward direction of the continuous web 2. Said continuous web 2 has a direction in which it traverses at least one section of said first rotary member 3 and is defined by said first direction V', and said continuous web 2 has a direction in which it traverses at least one section of said second rotary member 6 and is defined by said second direction V". In the event that said first direction V' and said second direction V" are opposite, said continuous web 2 traverses said first rotary member 3 and said second rotary member along a direction perpendicular to the forward direction of said continuous web 2; in other words, said continuous web 2, once having traversed at least one section of said first rotary member 3, is confined in the space defined by said first rotary member 3 and said second rotary member 6, assuming a direction perpendicular to the forward direction perpendicular at the input to said first rotary member, and sealed therein by said first sealing units 5' and said second sealing units 5".

According to a further aspect of the present invention, said method of sealing a continuous web is preferably intended for producing absorbent articles or for producing bags for containing liquid or solid products.

Purely by way of non-limiting example, said method finds advantageous application in the production of absorbent articles or for the production of containment bags for liquid or solid products.

Preferably, said absorbent products comprise nappies, adult diapers, sanitary pads, drawsheets and plasters.

Moreover, purely by way of non-limiting example, said method finds advantageous application in the production of containment bags for liquid or solid products for the food, pharmaceutical and tobacco industries.

Preferably, said method comprises a step of providing a first rotary member 3.

The term rotary member 3 refers to any member rotating in a respective direction (in this case, first direction V') about its own axis of rotation (in this case, 3a).

Preferably, said method comprises a step of providing one or more first support elements 4 of said continuous web 2.

The term support element 4 refers to any element configured to support said continuous web 2.

Preferably, said one or more first support elements 4 are supported by said first rotary member 3.

Preferably, said method comprises a step of providing at least one first sealing unit 5' for sealing the continuous web 2.

Preferably, said at least one first sealing unit is supported by said first rotary member 3.

Preferably, said method further comprises a step of providing a second member 6 rotating according to a second direction (V") and rotating about its own axis of rotation 6a.

Preferably, said method comprises a step of providing one or more second support elements 7 of said continuous web 2.

Preferably, said one or more support elements are supported by said second rotary member 6.

Preferably, said method comprises a step of providing at least one second sealing unit 5" for sealing the continuous web 2.

Preferably, said at least one second sealing unit is supported by said second rotary member 6.

Preferably, said first sealing unit 5' is configured to produce at least one first seal on said continuous web 2.

Preferably, said second sealing unit 5" is configured to perform at least one second seal on said continuous web 2.

Said at least one first seal and said at least one second seal can be made at the same sealing point, substantially overlapping each other.

In addition or alternatively, said at least one first seal and said at least one second seal can be produced at different sealing points, e.g. contiguous according to a certain pitch, substantially not overlapping each other.

These characteristics derive from the experimental tests carried out by the Applicant, in which the Applicant tried out different alternative solutions, in order to find the solution that best guaranteed high production flexibility and productivity.

In particular, the presence of said first rotary member 3 and of said second rotary member 6 with respective first sealing unit 5' and second sealing unit 5", respectively configured to perform at least one first seal and at least one second seal on said continuous web 2, allows to ensure high production flexibility and high productivity, since it avoids mechanical interference between sealing units of the same rotary member or adjacent rotary members, as well as the possibility of avoiding downtime in case of malfunction of at least one sealing unit, as it is possible to stop only one rotary member (supporting the malfunctioning sealing unit) while the further rotary member remains active.

Furthermore, in the event that said at least one first seal and said at least one second seal are performed at the same sealing point, substantially overlapping each other (in addition to or alternatively to being performed at different sealing points), the apparatus ensures high productivity; this is because, if one of said at least one first seal and said at least one second seal is performed according to sealing parameters that are lower than those required and predetermined (due, for example, to malfunctioning of a respective sealing unit), the other of said at least one first seal and said at least one second seal compensates for such failure and makes it possible to avoid rejection of the respective end product and/or stoppage of the respective rotary member (and of the production line).

According to a further aspect of the present invention, said first sealing units 5' and said second sealing units 5" are arranged respectively on said first rotary member 3 and said second rotary member 6 in such a manner that said first sealing units 5" and said second sealing units 5" can respectively perform in an alternating and complementary manner the seals provided on said continuous web 2.

In other words, said first sealing units 5' are arranged on said first rotary member 3 according to a predetermined configuration to produce said at least one first seal on said continuous web 2, while said second sealing units 5" are arranged on said second member 6 rotating according to a predetermined complementary configuration (i.e., alternating) with respect to the configuration for said first sealing units 5' so as to produce said at least one second seal on said continuous web 2 at said at least one first seal (as shown in Figure 2a) or at a sealing point contiguous to said at least one first seal (as shown in Figure 2b).

In this way, each rotary member has fewer (preferably, half the number of) sealing units required to produce said at least one first and at least one second sealing unit on said continuous web 2.

These characteristics derive from experimental tests carried out by the Applicant, wherein the Applicant tried out different alternative solutions, in order to identify the solution that best ensured high production flexibility and productivity.

In particular, this configuration for said first sealing units 5' and said second sealing units 5" ensures high production flexibility and high productivity, since it avoids mechanical interference between the sealing units of said rotary members, as well as the possibility of avoiding downtime in the event of malfunction of at least one sealing unit, since only one rotary member (supporting the malfunctioning sealing unit) can be stopped while the further rotary member remains active.

Furthermore, in the event that said at least one first seal and said at least one second seal are performed at the same sealing point, substantially overlapping each other (in addition to or alternatively to being performed at different sealing points), the apparatus ensures high productivity; this is because, if one of said at least one first seal and said at least one second seal is performed according to sealing parameters that are lower than those required and predetermined (due, for example, to malfunctioning of a respective sealing unit), the other of said at least one first seal and said at least one second seal compensates for such failure and makes it possible to avoid rejection of the respective end product and/or stoppage of the respective rotary member (and of the production line).

According to a further aspect of the present invention, each of said first sealing units 5' and said second sealing units 5" operates according to a pitch (i.e., a distance) equal to at least two products to be made on said continuous web 2.

This configuration for said first sealing units 5' and said second sealing units 5" ensures high production flexibility and high productivity, as it avoids mechanical interference between the sealing units of said rotary members.

According to a further aspect of the present invention, said at least one first seal and at least one second seal are made transverse to a long side L of said continuous web 2, as shown in Figure 2.

In particular, said transverse sealing is of the side seam bonding type.

According to a further aspect of the present invention, said continuous web 2 comprises at least one component of a sanitary absorbent item wearable as panties.

In other words, from said continuous web 2, by means of appropriate processing including said at least one first and at least one second seal, at least one component is obtained for the production of a sanitary absorbent item wearable as panties, preferably a pull-up nappy.

According to a further aspect of the present invention, said first sealing units 5' and/or said second sealing units 5" comprise an ultrasound sealing device or a thermomechanical sealing device or a thermal sealing device.

According to a further aspect of the present invention, said method comprises a step of providing actuation means M configured to vary a pitch defined by each of the one or more first support elements 4 and/or the one or more second support elements 7, expanding or contracting the working circumference of the first and/or second sealing units 5', 5".

In other words, said actuation means M are configured to radially vary the sealing pitch by acting on the mutual spacing between said one or more first support elements 4.

Alternatively or additionally, such actuation means M are configured to vary the sealing pitch radially by acting on the mutual spacing between said one or more second support elements 7.

This feature provides a high degree of production flexibility, as it avoids mechanical interference between the sealing units of said rotary members, allowing for continuous product change, size change or brand change.

According to a further aspect of the present invention, said actuation means M provided by said method comprise a control and command unit configured to adjust at least one mechanical element comprised by said one or more first support elements 4 and/or said one or more second support elements 7.

In other words, said control and command unit of said actuation means M comprises any automatic or semi-automatic technical solution configured to radially vary the sealing pitch by acting on the mutual spacing between said one or more first support elements 4.

Alternatively or additionally, said control and command unit (provided by said method) of said actuation means M comprises any automatic or semi-automatic technical solution configured to vary the sealing pitch radially by acting on the mutual spacing between said one or more second support elements 7.

This feature provides a high degree of production flexibility, as it avoids mechanical interference between the sealing units of said rotary members, allowing the product change, size change or brand change to be carried out continuously and efficiently, as no adjustments made manually by an operator are required (which could lead to problems in the event of human error).

According to a further aspect of the present invention, said method comprises a step of providing holding means configured to hold said continuous web 2 on said one or more first support elements 4 and/or one or more second support elements 7.

Preferably, said holding means comprise a belt and/or pneumatic vacuum.

Said characteristic ensures a high production efficiency, since by means of said holding means the continuous web 2 remains optimally positioned on said one or more first support elements 4 and/or said one or more second support elements 7, avoiding misplacements due to the high rotation speed of said at least one of said rotary members; thus, said first sealing units 5' and said second sealing units 5" can accurately and reproducibly provide said at least one first seal and said at least one second seal on said continuous web 2.

According to a further aspect of the present invention, said second rotary member 6 is arranged downstream of said first rotary member 3 and said second direction V" is concordant with said first direction V'.

In other words, said second rotary member 6 is arranged downstream of said first rotary member 3 along said forward direction of the continuous web 2. Said continuous web 2 has a direction in which it traverses at least one section of said first rotary member 3 and is defined by said first direction V', and said continuous web 2 has a direction in which it traverses at least one section of said second rotary member 6 and is defined by said second direction V". In the event that said first direction V' and said second direction V" are concordant, said continuous web 2 travels along said first rotary member 3 and said second rotary member along the forward direction of said continuous web 2.

According to an alternative embodiment not illustrated, said second rotary member 6 is arranged downstream of said first rotary member 3 and said second direction V" is opposite to said first direction V'.

In other words, said second rotary member 6 is arranged downstream of said first rotary member 3 along said forward direction of the continuous web 2. Said continuous web 2 has a direction in which it traverses at least one section of said first rotary member 3 and is defined by said first direction V', and said continuous web 2 has a direction in which it traverses at least one section of said second rotary member 6 and is defined by said second direction V". In the event that said first direction V' and said second direction V" are opposite, said continuous web 2 traverses said first rotary member 3 and said second rotary member along a direction perpendicular to the forward direction of said continuous web 2; in other words, said continuous web 2, once having traversed at least one section of said first rotary member 3, is confined in the space defined by said first rotary member 3 and said second rotary member 6, assuming a direction perpendicular to the forward direction perpendicular at the input to said first rotary member, and sealed therein by said first sealing units 5' and said second sealing units 5".

The apparatus 1 for sealing a continuous web, preferably for the production of absorbent articles or for the production of containment bags for liquid or solid products, and the related method, as described above, allow the above-mentioned purposes to be achieved, such as ensuring high production flexibility and high productivity.

It is finally clear that changes and variations can be made to the sealing apparatus 1, and to the related method herein described and illustrated without departing from the protection scope of the present invention, as defined in the appended claims.

## Claims

1. A rotary apparatus (1) for sealing a continuous web (2), preferably for producing absorbent items or for producing containment bags of liquid or solid products, comprising a first member (3) rotating according to a first direction (V') about an axis of rotation (3a) thereof, one or more first support elements (4) of said continuous web (2) supported by said first rotary member (3) and at least one first sealing unit (5') of the continuous web (2) supported by said first rotary member (3),
said rotary apparatus (1) furthermore comprising a second member (6) rotating according to a second direction (V") and rotating about an axis of rotation (6a) thereof, one or more second support elements (7) of said continuous web (2) supported by said second rotary member (6) and at least one second sealing unit (5") of the continuous web (2) supported by said second rotary member (6),
said first sealing unit (5') being configured to produce at least one first seal on said continuous web (2) and said second sealing unit (5") being configured to produce at least one second seal on said continuous web (2).

2. Apparatus according to claim 1, wherein said first sealing units (5') and said second sealing units (5") are placed respectively on said first rotary member (3) and said second rotary member (6) in such a way that said first sealing units (5') and said second sealing units (5") may respectively produce in an alternating and complementary way the seals provided on said continuous web (2).

3. Apparatus according to one or more of the previous claims, wherein each of said first sealing units (5') and said second sealing units (5") operates according to a pitch equal to at least two products on said continuous web (2).

4. Apparatus according to one or more of the previous claims, wherein said at least one first seal and at least one second seal are produced diagonally with respect to a long side (L) of said continuous web (2).

5. Apparatus according to one or more of the previous claims, wherein said continuous web (2) comprises at least one component of a sanitary absorbent item wearable as panties.

6. Apparatus according to one or more of the previous claims, wherein said first sealing units (5') and/or said second sealing units (5") comprise an ultrasound sealing device or a thermomechanical sealing device or a thermal sealing device.

7. Apparatus according to one or more of the previous claims, wherein said apparatus comprises actuation means (M) configured to vary a pitch defined by each one or more first support elements (4) and/or one or more second support elements (7).

8. Apparatus according to claim 7, wherein said actuation means (M) comprise a control and command unit configured to adjust at least one mechanical element comprised by said one or more first support elements (4) and/or one or more second support elements (7).

9. Apparatus according to one or more of the previous claims, wherein said apparatus comprises holding means configured to hold said continuous web (2) on said one or more first support elements (4) and/or one or more second support elements (7), said holding means comprising a belt and/or pneumatic vacuum.

10. Apparatus according to one or more of the previous claims, wherein said second rotary member (6) is placed downstream with respect to said first rotary member (3) and said second direction (V") is concordant with said first direction (V').

11. Method for sealing a continuous web (2), preferably for producing absorbent items or for producing containment bags of liquid or solid products, said method comprising the following steps:
- providing a first member (3) rotating according to a first direction (V') about an axis of rotation (3a) thereof;
- providing one or more first support elements (4) of said continuous web (2) supported by said first rotary member (3);
- providing at least one first sealing unit (5') of the continuous web (2) supported by said first rotary member (3);
- providing a second member (6) rotating according to a second direction (V") and rotating about an axis of rotation (6a) thereof;
- providing one or more second support elements (7) of said continuous web (2) supported by said second rotary member (6);
- providing at least one second sealing unit (5") of the continuous web (2) supported by said second rotary member (6),
said first sealing unit (5') being configured to produce at least one first seal on said continuous web (2) and said second sealing unit (5") being configured to produce at least one second seal on said continuous web (2).

12. Method according to claim 11, wherein said first sealing units (5') and said second sealing units (5") are placed respectively on said first rotary member (3) and said second rotary member (6) in such a way that said first sealing units (5') and said second sealing units (5") may respectively produce in an alternating and complementary way the seals provided on said continuous web (2).
